# DEMANDE DE BREVET EUROPEEN

(11) **EP 1 875 933 A1**
(43) Date de publication de la demande: **09.01.2008**
(21) Numéro de dépôt: 06116727.6
(22) Date de dépôt: 06.07.2006
(51) Int. Cl.: A61M 5/168

(54) **Dispositif médical pour l'administration d'une solution**

(71) Demandeur: DEBIOTECH S.A., 1004 Lausanne (CH)
(72) Inventeur: Neftel, Frédéric, 1005 Lausanne (CH)
(74) Mandataire: Grosfillier, Philippe

(57) **Abrégé**

Dispositif médical pour l'administration d'une solution, dispositif comprenant des moyens de pompage, un réservoir, des moyens de communication entre ledit réservoir et lesdits moyens de pompage, ainsi qu'une ligne patient adaptée pour relier le dispositif médical à un patient, caractérisé par le fait qu'il comprend des moyens pour déterminer les caractéristiques physico-chimiques de la solution en fonction du temps au moyen d'un modèle mathématique.

## Description

### Domaine de l'invention

La présente invention se situe dans le domaine de l'administration de solutions médicales, par exemple de solutions contenant de l'insuline.

### Etat de la technique

Il existe plusieurs types de dispositifs médicaux destinés à l'administration d'une solution. De tels dispositifs sont constitués d'au moins un réservoir communiquant avec une pompe, le tout étant relié à un patient au moyen d'une tubulure ou d'un accès sous-cutané, intra-musculaire ou veineux..

La solution qui est contenue dans le réservoir est administrée en général sur une certaine durée ou à intervalles déterminés.

Un problème constaté avec les dispositifs de l'état de la technique résulte du fait que la concentration de la solution peut varier au cours du temps du fait de son stockage dans le réservoir. Voir à cet effet le graphe ci-joint qui montre la variation pendant 35 jours de la concentration d'insuline dans un réservoir de type "Topas" qui comporte sur l'une de ses parois un film souple constitué de surlin à l'intérieur et mylar à l'extérieur, la solution d'insuline étant conservée à 37°C

Les causes de cette variation peuvent être l'évaporation que subit la solution, la stabilité du médicament ou toute altération et/ou modification de son contenu.

Ce phénomène est d'autant plus accentué lorsque les parois du réservoir sont perméables et/ou que la durée d'administration de la solution est longue.

L'administration d'une solution dont la concentration et ou les caractéristiques varient au cours du temps peut provoquer des complications, voire une menace pour le traitement ou la santé du patient.

Il existe donc un besoin de remédier aux problèmes précités

### Description de l'invention

La présente invention constitue une amélioration par rapport aux dispositifs de l'état de la technique.

Dans l'invention, la solution du problème précité consiste en un dispositif médical pour l'administration d'une solution qui comprend des moyens de pompage, un réservoir, des moyens de communication entre ledit réservoir et lesdits moyens de pompage, ainsi qu'une ligne patient adaptée pour relier le dispositif médical à un patient. Le dispositif selon l'invention se caractérise par le fait qu'il comprend des moyens pour déterminer les caractéristiques physico-chimiques de la solution en fonction du temps au moyen d'un modèle mathématique.

Selon un mode de réalisation de l'invention, le dispositif comprend des moyens pour faire varier l'administration de la solution en fonction d'une variation de la concentration de la solution déterminée au moyen d'un modèle mathématique.

Avantageusement, le modèle mathématique peut notamment tenir compte des caractéristiques d'évaporation, d'absorption, d'adsorption et ou toute autre modification de la solution au cours du temps en fonction de l'exposition à la température, à l'humidité, à la pression et à toute autre condition d'utilisation, ainsi qu'éventuellement des conditions de vieillissement et/ou altération du réservoir au cours du temps. Ainsi les caractéristiques de la solution peuvent être calculée en fonction de ce modèle mathématique à tout instant.

Dès lors que les caractéristiques de la solution sont modifiées, il est souhaitable d'en tenir compte dans le mode d'administration de la solution afin d'assurer une administration efficace et sure.

Il peut notamment s'agir d'une évaporation de l'eau au travers d'une membrane souple constituant le réservoir au cours du temps, qui a pour conséquence d'augmenter la concentration du principe actif dans la solution. Dans ce cas, une absence de correction de la concentration aurait pour effet de surdoser le médicament administré. Le principe de l'invention est de corriger l'administration de la solution afin d'assurer une quantité toujours efficace de principe actif en utilisant le modèle mathématique pour tenir compte de la concentration effective de la solution au cours du temps et adapter le débit en conséquence. Cette évaporation étant souvent fonction de la température, il peut être utile d'intégrer également dans le modèle mathématique l'effet de l'exposition à la température au cours du temps afin d'être à même de prévoir au plus juste la concentration probable de la solution à un instant donné. Pour cela, il est nécessaire de disposer d'un capteur de température, de préférence en continu, le modèle mathématique étant à même d'intégrer le calcul de l'évaporation au cours du temps selon un modèle qui peut être non-linéaire.

Il peut également être utile d'utiliser le modèle mathématique afin de prévoir à tout instant la quantité de certaines substances contenues dans le réservoir, tels que des conservateurs (métacrésol, phénol) très souvent utilisés en association avec certaines substances médicales (insuline par exemple). Dès lors que la teneur dans certaines de ces substances n'est plus suffisante, il peut être utile de prévenir l'utilisateur qu'un re-remplissage du réservoir est nécessaire ou que le réservoir doit être changé. Certaines de ces substances étant très volatiles, elles ont tendance à être les premières à diminuer dans le réservoir (ex : phénol).

Selon un mode de réalisation de l'invention, il peut être utile de mesurer certains paramètres de la solution dont l'évolution peut servir d'indicateur au modèle mathématique afin de corriger le calcul des caractéristiques de la solution. Il peut, par exemple, s'agir de la conductivité de la solution qui est un bon indicateur de l'évaporation de l'eau au cours du temps.

Selon un mode de réalisation de l'invention, l'utilisateur peut lire en continu des informations relatives aux caractéristiques de la solution contenue dans le réservoir. Une telle indication peut, par exemple, être représentative de la qualité de la solution (Excellente, Moyenne, Limite), afin de lui permettre de prévoir un re-remplissage de la solution et/ou changement de réservoir à l'avance. Une telle indication est très différente de la seule indication aujourd'hui disponible sur les dispositifs d'administration, à savoir le volume résiduel de la solution et offre une plus grande sécurité à l'utilisateur.

La présente invention présente plusieurs avantages, en particulier d'assurer à tout moment que la dose administrée au patient est la plus correcte possible, compte tenu des modifications prédictives des caractéristiques de la solution au cours du temps. En outre, elle permet d'assurer que lorsque les conditions d'efficacité et/ou de sécurité ne sont plus assurées, l'utilisateur est avertit et le réservoir est changé ou re-remplis à temps. Enfin elle permet également de prévenir l'utilisateur suffisamment à l'avance d'un re-remplissage ou changement nécessaire du réservoir.

Il peut être également important, dans le cas d'un re-remplissage du réservoir, de tenir compte du volume résiduel éventuel de solution présente au moment du re-remplissage (dans le cas ou cette solution résiduelle n'est pas vidée) afin de corriger les caractéristiques de la nouvelle solution obtenue après re-remplissage en fonction de la dilution réalisée entre l'ancienne et la nouvelle solution, toujours dans le but d'assurer une meilleure efficacité et sécurité dans l'administration effective de la solution au patient.

Le graphe joint à la présente description montre l'évolution de la concentration de différents composants d'une solution d'insuline U100 (Novorapid) au cours du temps dans un réservoir comprenant une partie rigide en Topas 8007S-04 de Topas Advanced Polymers GmbH, et un film souple constitué de Surlyn 1702 (30 µm) à l'intérieur et de Mylar D820 (12 µm) à l'extérieur, les deux matériaux en provenance de la société Dupont et la solution étant maintenue à température constante (37°C) pendant 35 jours.

Les résultats observés dans le temps, en fonction de la température, relativement à la concentration d'insuline et des différents agents conservateurs sont également indiqués dans les tables suivantes pour des conditions différentes d'évaluation:
A) Etude insuline U100 Novorapid dans 3 poches différentes sur 4 semaines à 35°C avec et sans vibration de la poche :

| Perte totale d'eau (%) | | | | | |
|---|---|---|---|---|---|
| Type de poche | **1** | **3** | **4** | Moyenne | écart type |
| Eau (poids total) | 2.132 | 1.985 | 2.116 | | |
| 02.06.2006 | 95% | 95% | 94% | **94%** | 1% |
| 09.06.2006 | 93% | 93% | 91 % | **92%** | 1% |
| 16.06.2006 | 91 % | 91 % | 89% | **90%** | 1% |
| 23.06.2006 | 89% | 89% | 86% | **88%** | 1% |
| | | | | | |
| **Phenol** | | | | | |
| 02.06.2006 | 99% | 97% | 99% | **98%** | 1% |
| 09.06.2006 | 97% | 96% | 101% | **98%** | 3% |
| 16.06.2006 | 98% | 98% | 100% | **99%** | 1% |
| 23.06.2006 | 97% | 97% | 99% | **98%** | 1% |
| | | | | | |
| **m-Cresol** | | | | | |
| 02.02.2006 | 87% | 85% | 87% | **86%** | 1% |
| 09.06.2006 | 81% | 80% | 84% | **81%** | 2% |
| 16.06.2006 | 75% | 74% | 78% | **76%** | 2% |
| 23.06.2006 | 68% | 69% | 71 % | **69%** | 2% |
| | | | | | |
| **Insulin** | | | | | |
| 02.02.2006 | 103% | 102% | 105% | **103%** | 2% |
| 09.06.2006 | 109% | 106% | 109% | **108%** | 2% |
| 16.06.2006 | 111% | 110% | 114% | **112%** | 2% |
| 23.06.2006 | 119% | 117% | 123% | **120%** | 3% |

B) Etude insuline U100 Novorapid dans 5 poches différentes (A,B, D, E et F) sur 1 à 6 semaines à différentes températures (4°C et 35°C) avec (vibr) et sans (still) vibration de la poche en comparaison avec de l'insuline contenue dans une carpule (catridge) :

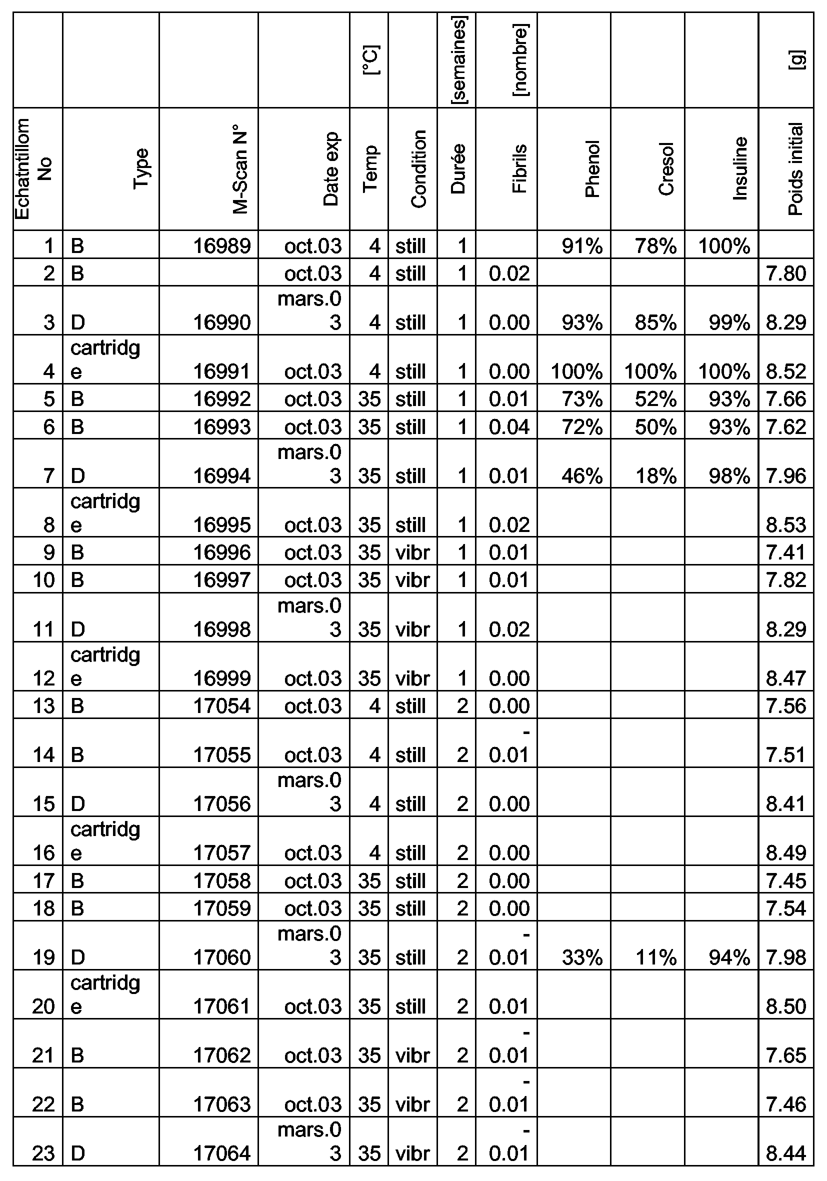

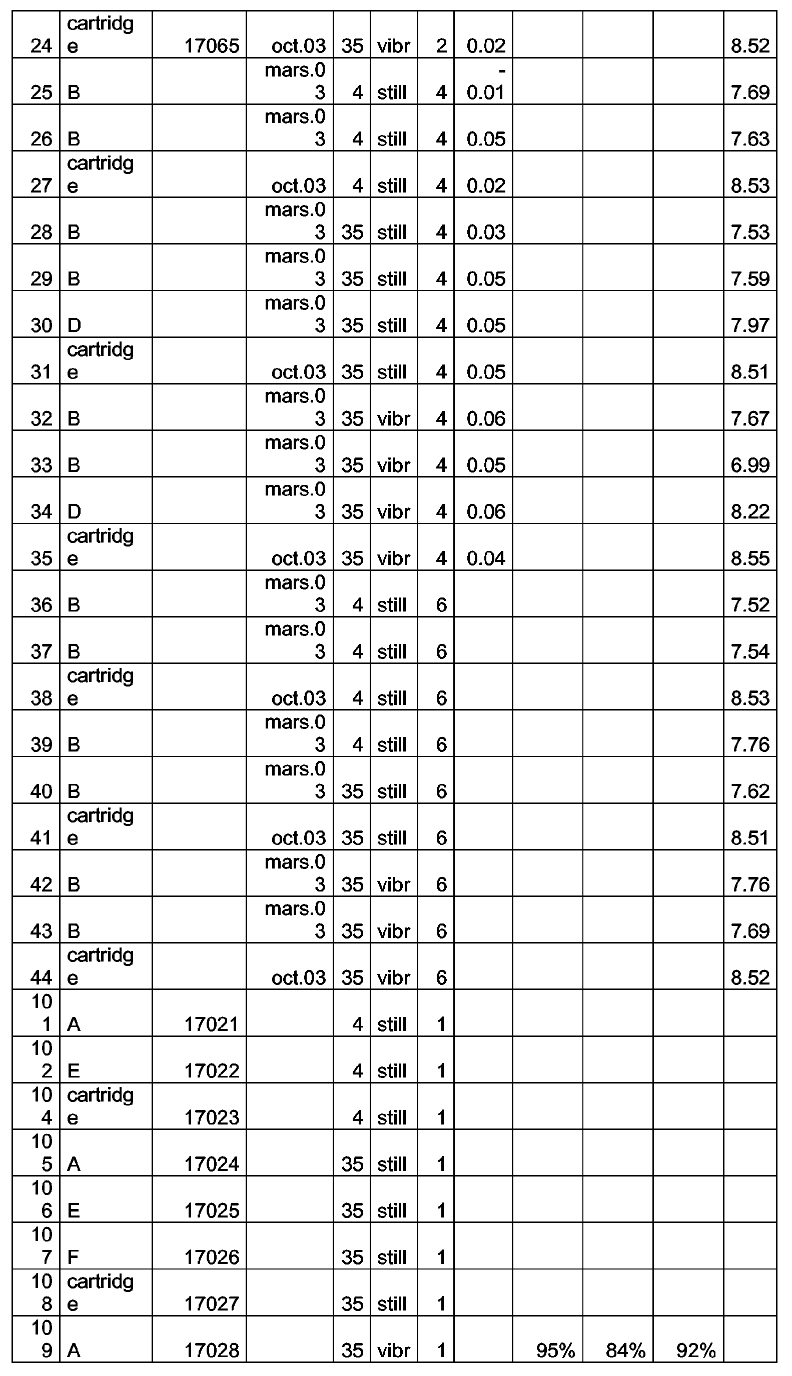

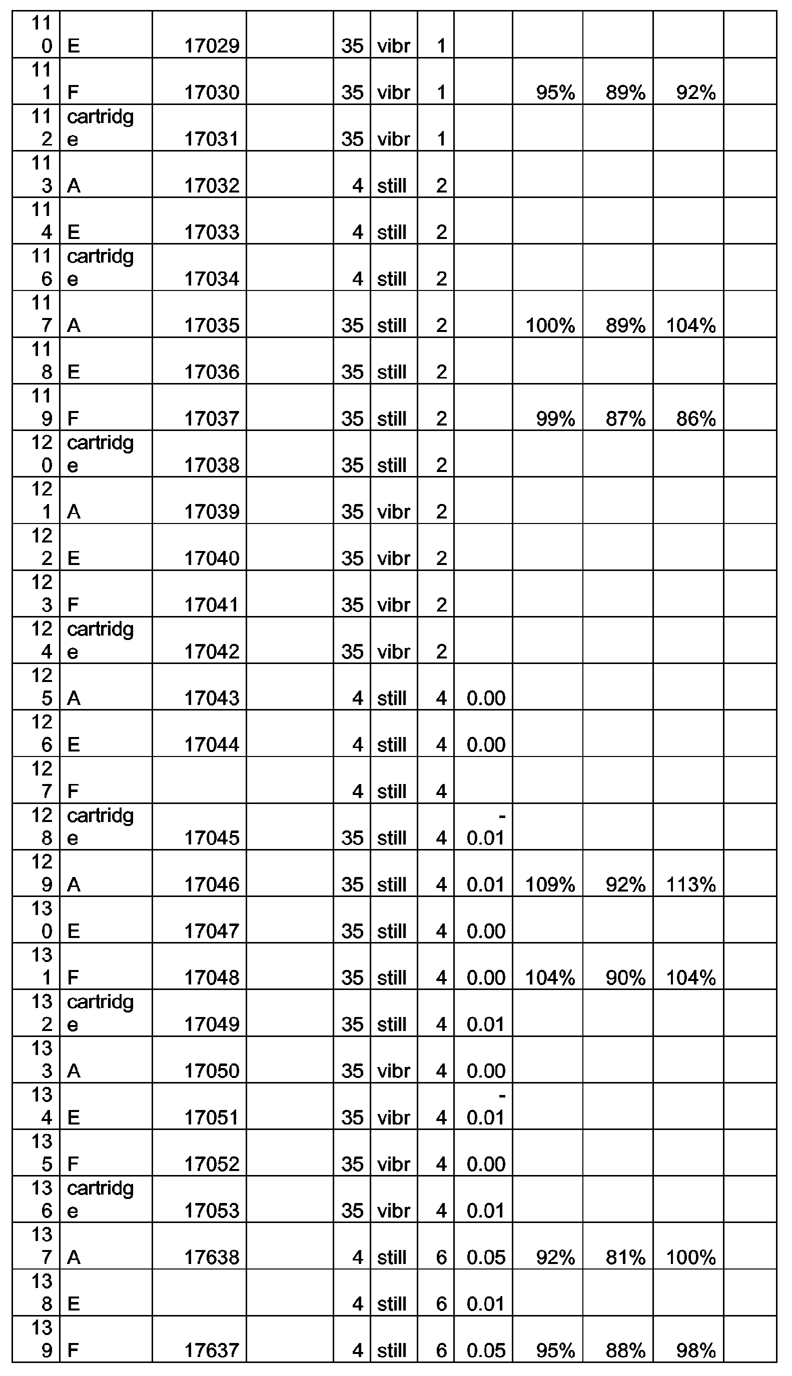

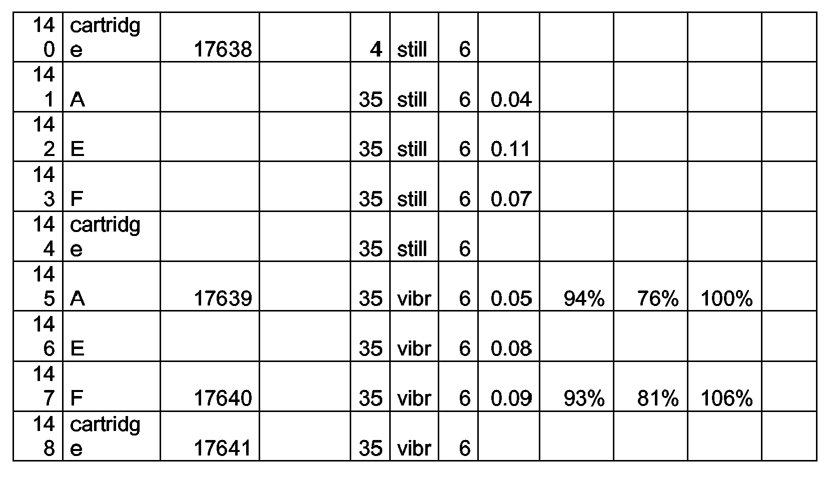

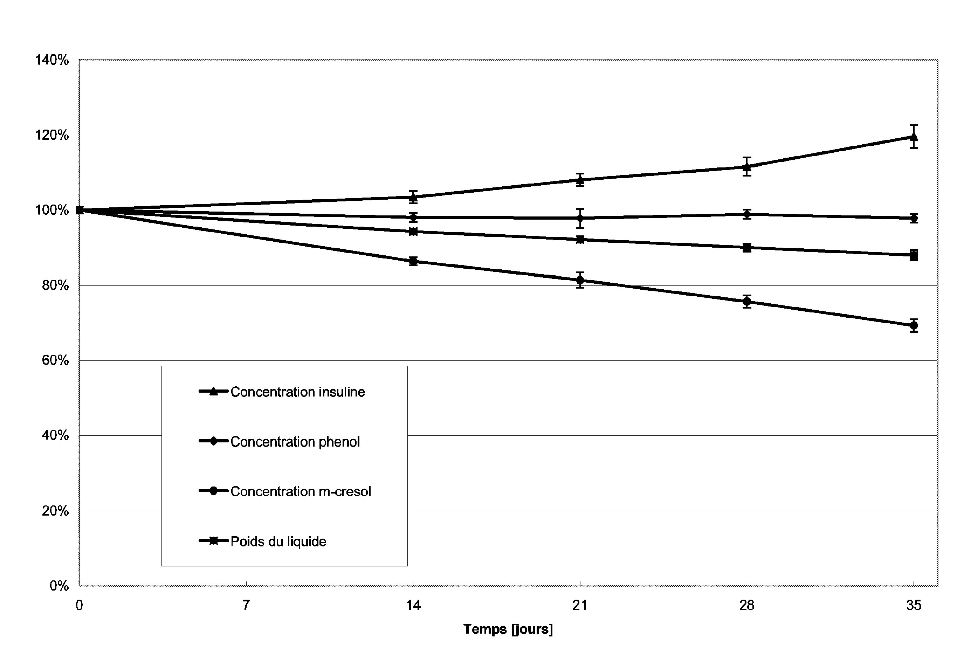

## Revendications

1. Dispositif médical pour l'administration d'une solution, dispositif comprenant des moyens de pompage, un réservoir, des moyens de communication entre ledit réservoir et lesdits moyens de pompage, ainsi qu'une ligne patient adaptée pour relier le dispositif médical à un patient, **caractérisé par le fait qu'**il comprend des moyens pour déterminer les caractéristiques physico-chimiques de la solution en fonction du temps au moyen d'un modèle mathématique.

2. Dispositif selon la revendication 1 , **caractérisé par le fait qu'**il comprend des moyens pour faire varier l'administration de la solution en fonction d'une variation de la concentration de la solution déterminée au moyen du modèle mathématique.

3. Dispositif l'une quelconque des revendications précédentes dans lequel ledit modèle mathématique tient compte de la perméabilité des parois du réservoir.

4. Dispositif selon l'une quelconque des revendications précédentes dans lequel ledit modèle mathématique tient compte de l'évolution de la température au cours du temps.

5. Dispositif selon la revendication 4 comprenant des moyens pour mesurer régulièrement la température de la solution, lesdits moyens pour faire varier l'administration de la solution étant reliés fonctionnellement aux dits moyens pour mesurer en continu la température.

6. Dispositif l'une quelconque des revendications précédentes dans lequel ledit modèle mathématique tient compte de la perméabilité des parois du réservoir en fonction du temps et de la température.

7. Dispositif l'une quelconque des revendications précédentes dans lequel ledit modèle mathématique tient compte de la dégradation de la solution en fonction du temps et/ou de la température.

8. Dispositif selon la revendication 7 , **caractérisé par le fait qu'**il comprend des moyens pour faire varier l'administration de la solution en fonction de la concentration de substance active non-dégradée contenue dans le réservoir.

9. Dispositif selon l'une quelconque des revendications précédentes dans lequel le modèle mathématique est adapté pour déterminer la variation de la nouvelle concentration de la solution après chaque re-remplissage du réservoir en fonction du volume résiduel de la solution et le volume du re-remplissage de la nouvelle solution.

10. Dispositif selon l'une quelconque des revendications précédentes dans lequel le modèle mathématique est adapté pour déterminer les caractéristiques de la solution après chaque re-remplissage du réservoir,

11. Dispositif selon l'une quelconque des revendications précédentes comprenant des capteurs pour mesurer certaines caractéristiques de la solution, lesdits capteurs fournissant une information utilisée par le modèle mathématique.

12. Dispositif selon la revendication 11 dans lequel ledit capteur est un capteur qui mesure la conductivité et/ou la résistance de la solution, le ph ou toute autre mesure physique de la solution.

13. Dispositif selon l'une quelconque des revendications précédentes comprenant une première alarme adaptée pour se déclencher lorsque la solution contenue dans le réservoir est estimée ne plus correspondre aux exigences nécessaires au traitement.

14. Dispositif selon la revendication 13 dans lequel l'alarme a pour but de prévenir l'utilisateur qu'un re-remplissage du réservoir doit être effectué.

15. Dispositif selon l'une quelconque des revendications précédentes comprenant une deuxième alarme adaptée pour se déclencher lorsque la température de la solution contenue dans le réservoir a dépassé un seuil déterminé pendant une durée minimale.

16. Dispositif selon l'une quelconque des revendications précédentes comprenant une troisième alarme adaptée pour se déclencher en fonction d'un modèle mathématique qui tient compte de la température à laquelle est exposée la solution à chaque instant et de la durée de cette exposition.

17. Dispositif selon l'une quelconque des revendications précédentes comprenant un réservoir adapté pour contenir de l'insuline.

18. Dispositif selon l'une quelconque des revendications précédentes dans lequel le réservoir comprend au moins une membrane non totalement imperméable à la vapeur d'eau et/ou à certains composés de la solution.

19. Dispositif selon l'une quelconque des revendications précédentes dans lequel le réservoir et les moyens de pompage sont assurés par une seringue dont les parois ne sont pas totalement imperméable à la vapeur d'eau et/ou à certains composés de la solution.

20. Dispositif selon l'une quelconque des revendications précédentes comprenant des moyens pour indiquer le temps restant d'utilisation possible de la solution dans les conditions d'utilisation courantes et/ou prévisibles.

21. Dispositif selon l'une quelconque des revendications précédentes dans lequel le dispositif médical d'injection est adapté pour informer l'utilisateur de la qualité de la solution selon un mode qui lui permet de prévoir un changement et/ou un re-remplissage à l'avance.
